# EUROPEAN PATENT SPECIFICATION

(11) **EP 3 243 502 B1**
(45) Date of publication and mention of the grant of the patent: **25.12.2019**
(21) Application number: 15875677.5
(22) Date of filing: 29.12.2015
(51) Int. Cl.: A61K 8/81, A61K 8/72, A61K 8/34, A61K 8/19, A61K 8/06, A61Q 19/00, A61K 8/898, A61K 8/02, A61K 8/11, A61K 8/20, C08F 112/08, C08J 3/12, C08F 257/02

(54) **CHEMICALLY ASYMMETRIC ANISOTROPIC POWDER AND WATER-IN-OIL (W/O) EMULSIFICATION COMPOSITION CONTAINING SAME**
CHEMISCH ASYMMETRISCHES ANISOTROPES PULVER UND WASSER-IN-ÖL-EMULGIERUNGSZUSAMMENSETZUNG DAMIT
POUDRE ANISOTROPE CHIMIQUEMENT ASYMÉTRIQUE ET COMPOSITION D'ÉMULSIFICATION EAU-DANS-HUILE (W/O) CONTENANT CETTE DERNIÈRE

(30) Priority: 31.12.2014 KR 20140194547; 28.12.2015 KR 20150187740
(43) Date of publication of application: 15.11.2017
(73) Proprietor: Amorepacific Corporation, Seoul 140-777 (KR)
(72) Inventor: SHIN, Ji Sik, Yongin-si Gyeonggi-do 17074 (KR); NAM, Jin, Yongin-si Gyeonggi-do 17074 (KR); PARK, Sung Il, Yongin-si Gyeonggi-do 17074 (KR); AN, Soon Ae, Yongin-si Gyeonggi-do 17074 (KR); KANG, Byung Young, Yongin-si Gyeonggi-do 17074 (KR); HAN, Sang Hoon, Yongin-si Gyeonggi-do 17074 (KR)
(74) Representative: Agasse, Stéphane
(86) International application number: PCT/KR2015/014415
(87) International publication number: WO 2016/108583

(56) References cited:
- EP-A1- 3 150 191
- WO-A1-2015/183042
- WO-A1-2015/183042
- KR-A- 20090 073 368
- KR-A- 20140 073 211
- KR-A- 20140 091 556
- KR-B1- 101 299 148
- Jason D Forster: "Self-Assembly of Photonic Materials Beyond Crystals of Spheres: Amorphous Close-Packed Spheres and Crystalline Dumbbells", , 15 September 2012 (2012-09-15), XP055431896, ISBN: 978-1-267-57392-6 Retrieved from the Internet: URL:http://epo.summon.serialssolutions.com /2.0.0/link/0/eLvHCXMwtV3NS8MwFA-KF_GiKPgJ OehJCk36kcab-3BDEKabB08jbRJ22CxsE_S_9722aT sHggcvjzZpQ8mvbX7vI-8Rci1NBqzWSk-rRHphoOGb i3ztMeuHzGrjW41Iv3XDyUC8DLBag0uT07T9K9LQBl jjztk_oF0PCg1wDJiDBNRB_iDEW670am-HmVsPfbmL dF64z0ezfF3UuXlS6_IJbqt9K93lF5DDMn_yGPMLlA Fy94scZh9j [retrieved on 2017-12-05]
- JIN-GYU PARK ET AL: "High-Yield Synthesis of Monodisperse Dumbbell-Shaped Polymer Nanoparticles", JOURNAL OF THE AMERICAN CHEMICAL SOCIETY, vol. 132, no. 17, 5 May 2010 (2010-05-05), pages 5960-5961, XP055385995, US ISSN: 0002-7863, DOI: 10.1021/ja101760q
- JIN WOONG KIM , DAEYEON LEE , HO CHEUNG SHUM , DAVID A. WEITZ: "Colloid Surfactants for Emulsion Stabilization", ADVANCED MATERIALS, vol. 20, no. 17, 17 July 2008 (2008-07-17) , pages 3239-3243, XP002782783, DOI: 10.1002/adma.200800484

## Description

### [Technical Field]

The present disclosure relates to a water-in-oil (W/O) type emulsion comprising chemically asymmetric anisotropic powder.

### [Background Art]

Various methods for preparing fine particles (nano-size, micro-size, etc.) having different shapes and sizes have been reported. In the following documents from Jason D Forster: "Self-Assembly of Photonic Materials Beyond Crystals of Spheres: Amorphous Close-Packed Spheres and Crystalline Dumbbells", 15 September 2012 (ISBN: 978-1-267-57392-6) and JIN-GYU PARK ET AL: "High-Yield Synthesis of Monodisperse Dumbbell-Shaped Polymer Nanoparticles", JOURNAL OF THE AMERICAN CHEMICAL SOCIETY, vol. 132, no. 17, 5 May 2010, pages 5960-5961, amphiphilic anisotropic powders have been synthesized. Particularly, spherical microparticles including polymers have a size and shape controllable according to preparation methods thereof, and thus have high applicability. For example, there is provided a Pickering emulsion which uses spherical microparticles to form stabilized macroemulsion particles.

Such a Pickering emulsion using solid spherical powder forms a W/O emulsion or O/W emulsion depending on the wettability at the solid powder interface, i.e., depending on the degree of oleophilicity or hydrophilicity. As a factor determining the orientability of a surface film, there is a contact angle. When a contact angle is smaller than 90°, a larger part of particle surfaces is present as an aqueous phase to form an O/W emulsion. Meanwhile, when a contact angle is larger than 90°, a larger part of particle surfaces is present as an oil phase to form a W/O emulsion.

In general, a water-in-oil (W/O) formulation has a very limited composition of emulsion for providing various feelings of use due to the problem of stability during refrigeration/thawing of emulsion particles. To stabilize a W/O formulation, polyol/salt/alcohol or the like has been used for an inner phase. However, such an inner phase composition affects the emulsion interface and thickening system, and thus causes a limitation in emulsion formulation to be made. In addition, an inorganic thickener (e.g. bentone) has been used for an outer phase in an excessive amount to stabilize an emulsion composition. However, in this case, there are problems of an increase in viscosity and a limitation in feeling of use.

### [Disclosure]

### [Technical Problem]

The technical problem to be solved by the present disclosure is to provide a water-in-oil type emulsion composition not using a conventional aqueous emulsifier but using chemically asymmetric anisotropic powder to ensure emulsion stability, and having various emulsion composition ratios to allow variations in feeling of use, such as controlled viscosity.

### [Technical Solution]

In one general aspect, there is provided a water-in-oil (W/O) type emulsion comprising an asymmetric anisotropic powder which includes a first hydrophilic polymer spheroid and a second hydrophobic polymer spheroid, wherein the first and second polymer spheroids are bound to each other with a structure in which one polymer spheroid at least partially infiltrates the other polymer spheroid; the first polymer spheroid has a core-shell structure; and the shell has a functional group.

The second polymer spheroid and the core of the first polymer spheroid include a vinyl polymer, and the shell of the first polymer spheroid may include a copolymer of a vinyl monomer with a functional group-containing monomer; and the vinyl polymer is polystyrene, and wherein the aqueous phase part of the water-in-oil type emulsion composition comprises a salt.

According to still another embodiment, the functional group may be siloxane.

According to yet another embodiment, the chemically asymmetric anisotropic powder may have an asymmetric snowman shape or asymmetric reverse snowman shape.

According to an embodiment, the chemically asymmetric anisotropic powder may be present in an amount of 1-15 wt% based on the total weight of the emulsion composition.

According to still another embodiment, the water-in-oil type emulsion composition may include an alcohol.

According to still another embodiment, the salt may be at least one selected from the group consisting of sodium chloride, potassium chloride, lithium chloride, calcium chloride and magnesium chloride.

According to still another embodiment, the salt may be present in an amount of 0.01-10 wt% based on the total weight of the water-in-oil type emulsion composition.

According to still another embodiment, the water-in-oil type emulsion composition may include the chemically asymmetric anisotropic powder, oil phase part and aqueous phase part at a mixing ratio of 1-15:50-80:10-30 on the weight basis.

According to still another embodiment, the water-in-oil type emulsion composition may include emulsion particles having a size of 2-200 µm.

According to yet another embodiment, the water-in-oil type emulsion composition may have a formulation with a low viscosity of 10000 cps or less.

### [Advantageous Effects]

According to the embodiments of the present disclosure, there is provided chemically asymmetric anisotropic powder which forms a strong binding between powder particles to form a stabilized interface film by virtue of its geometrically linear powder characteristics.

In addition, according to the embodiments of the present disclosure, there is provided a water-in-oil type emulsion composition which includes the chemically asymmetric anisotropic powder and is stabilized against various inner/outer compositions.

In addition, according to the embodiments of the present disclosure, there is provided a water-in-oil type emulsion composition having a low-viscosity formulation. The emulsion composition includes chemically asymmetric anisotropic powder forming different sizes of emulsion particles and has a significantly lighter feeling of use as compared to the conventional W/O formulations.

### [Description of Drawings]

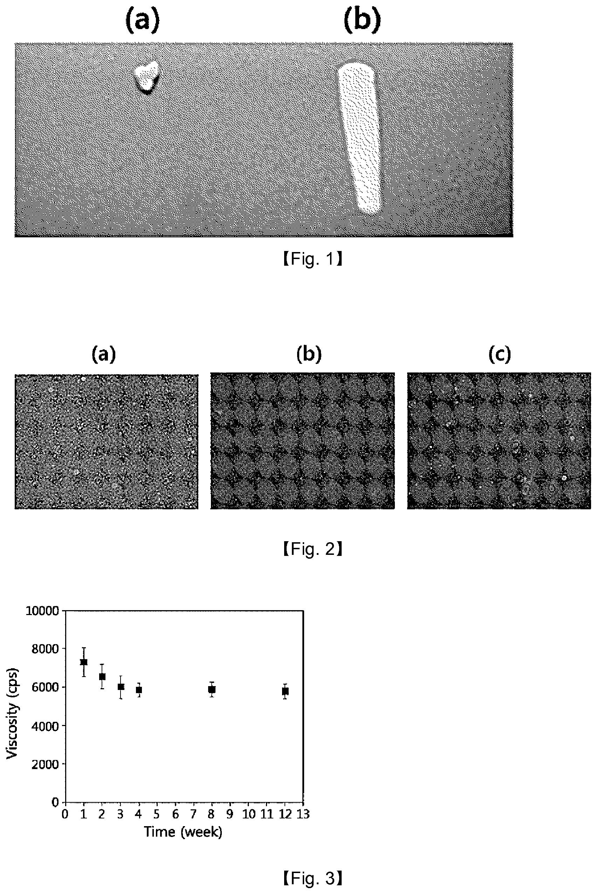

Fig. 1 shows images illustrating the viscosity of the water-in-oil type emulsion composition according to Example 1 as compared to that of the water-in-oil type emulsion composition according to Comparative Example 1, wherein (a) shows a conventional W/O formulation according to Comparative Example 1, and (b) shows a W/O formulation including anisotropic powder according to Example 1.
Fig. 2 shows an image illustrating the emulsion particles in the water-in-oil type emulsion composition according to Example 1, wherein (a) shows the emulsion particles at room temperature right after the preparation, (b) shows the emulsion particles after storing them under refrigeration for 2 months, and (c) shows the emulsion particles after storing them for 2 months while repeating refrigerating and thawing four times.
Fig. 3 is a graph illustrating variations in viscosity with time in the water-in-oil type emulsion composition according to Example 1.

### [Best Mode]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these embodiments are provided so that this disclosure will be thorough and complete, and will fully convey the scope of the present disclosure to those skilled in the art. In the drawings, the shape, size and regions, and the like, of the drawing may be exaggerated for clarity. In addition, although a part of constitutional elements is shown for convenience of description, the remaining part may be understood with ease by those skilled in the art. Further, it will be understood by those skilled in the art that various changes in form and details may be made thereto without departing from the disclosure as defined by the appended claims.

As used herein, "substituted" means that at least one hydrogen atom of the functional group described herein is substituted with a halogen atom (F, CI, Br or I), hydroxyl group, nitro group, imino group (=NH, =NR, wherein R is a C1-C10 alkyl group), amidino group, hydrazine or hydrazine group, carboxyl group, substituted or non-substituted C1-C20 alkyl group, substituted or non-substituted C3-C30 heteroaryl group, or substituted or non-substituted C2-C30 heterocycloalkyl group, unless otherwise stated.

As used herein, "(meth)acryl" means acryl and/or methacryl.

As used herein, the particle size of amphiphilic anisotropic powder is measured as the maximum length that is the largest length of the powder particles. As used herein, the particle size range of amphiphilic anisotropic powder means that at least 95% of the amphiphilic anisotropic powder present in a composition belongs to the corresponding range.

As used herein, the average particle diameter of emulsion particles means the average of diameter of each particle. As used herein, the average particle diameter range of emulsion particles means that at least 95% of the emulsion particles present in a composition belongs to the corresponding range.

In one aspect, there is provided chemically asymmetric anisotropic powder which includes a first hydrophilic polymer spheroid and a second hydrophobic polymer spheroid, wherein the first and second polymer spheroids are bound to each other with a structure in which one polymer spheroid at least partially infiltrates the other polymer spheroid; the first polymer spheroid has a core-shell structure; and the shell has a functional group.

As used herein, a spheroid means a single body formed of polymers. For example, it may have a spherical or ellipsoidal shape and a micro-scale or nano-scale long axis length based on the largest length in the section of the body.

According to an embodiment, the second polymer spheroid and the core of the first polymer spheroid include a vinyl polymer, and the shell of the first polymer spheroid may include a copolymer of a vinyl polymer with a functional group-containing monomer.

The vinyl polymer is polystyrene.

According to still another embodiment, the functional group may be siloxane.

According to still another embodiment, at least one of the first polymer spheroid and the second polymer spheroid may include an ionic vinyl polymer.

According to still another embodiment, the ionic vinyl polymer may be a sodium 4-vinylbenzene sulfonate polymer.

According to still another embodiment, the functional group-containing monomer may be a siloxane-containing (meth)acrylate, and particularly it may be 3-(trimethoxysilyl)propyl acrylate, 3-(trimethoxysilyl)propyl methacrylate, vinyltriethoxysilane, vinyltrimethoxysilane or a mixture thereof.

According to still another embodiment, the shell of the first polymer spheroid may further include a hydrophilic functional group introduced thereto.

According to still another embodiment, the hydrophilic functional group may be a negatively charged or positively charged functional group or polyethylene glycol (PEG)-based functional group, and may be at least one selected from the group consisting of carboxylic acid group, sulfone group, phosphate group, amino group, alkoxy group, ester group, acetate group, polyethylene glycol group and hydroxyl group.

The geometrically dumbbell-like chemically asymmetric anisotropic powder allows preparation of a stabilized water-in-oil type emulsion composition by virtue of its strong emulsification capability. The chemically asymmetric anisotropic powder forms macroemulsion particles and makes the interface physically strong, and thus produces larger emulsion particles as compared to the conventional W/O or W/S (silicon) formulations. Therefore, it is possible to realize low viscosity and to prevent coalescence of emulsion particles. According to still another embodiment, the chemically asymmetric anisotropic powder may have an asymmetric snowman shape or asymmetric reverse snowman shape.

According to yet another embodiment, the chemically asymmetric anisotropic powder may have a particle size of 100-1500 nm. In a variant, the chemically asymmetric anisotropic powder may have a particle size of 100-500 nm, or 200-300 nm. Herein, the particle size means the largest length of the amphiphilic powder. Particularly, the chemically asymmetric anisotropic powder may have a particle size of at least 100 nm, at least 200 nm, at least 300 nm, at least 400 nm, at least 500 nm, at least 600 nm, at least 700 nm, at least 800 nm, at least 900 nm, at least 1000 nm, at least 1100 nm, at least 1200 nm, at least 1300 nm or at least 1400 nm, and at most 1500 nm, at most 1400 nm, at most 1300 nm, at most 1200 nm, at most 1100 nm, at most 1000 nm, at most 900 nm, at most 800 nm, at most 700 nm, at most 600 nm, at most 500 nm, at most 400 nm, at most 300 nm or at most 200 nm.

In another aspect, there is provided a water-in-oil (W/O) type emulsion composition including the chemically asymmetric anisotropic powder.

According to an embodiment, the chemically asymmetric anisotropic powder may be present in an amount of 1-15 wt% based on the total weight of the water-in-oil type emulsion composition. According to another embodiment, the chemically asymmetric anisotropic powder may be present in an amount of 0.5-5 wt% based on the total weight of the emulsion composition. Particularly, the chemically asymmetric anisotropic powder may be present in an amount of at least 0.1 wt%, at least 0.5 wt%, at least 1 wt%, at least 2 wt%, at least 4 wt%, at least 6 wt%, at least 8 wt%, at least 10 wt% or at least 12 wt%, and at most 15 wt%, at most 12 wt%, at most 10 wt%, at most 8 wt%, at most 6 wt%, at most 4 wt%, at most 2 wt%, at most 1 wt% or at most 0.5 wt%. It is possible to control the size of emulsion particles from several micrometers to several tens or several hundreds of micrometers by adjusting the content of chemically asymmetric anisotropic powder.

According to still another embodiment, the water-in-oil type emulsion composition may include an alcohol. The chemically asymmetric anisotropic powder may be dispersed in an alcohol, and then added together with an aqueous phase part to provide a water-in-oil type emulsion composition. As an alcohol, a C1-C4 lower alcohol, polyol or a mixture thereof may be used. It is possible to lower the freezing point of water by using a C1-C4 lower alcohol, polyol or a mixture thereof, and thus to prevent degradation of stability against refrigeration/thawing caused by formation of macroemulsion particles and to increase stability.

The C1-C4 lower alcohol may be at least one selected from the group consisting of methanol, ethanol and butanol, particularly ethanol.

The polyol may be a polyhydric alcohol, i.e., an aliphatic compound having at least two hydroxyl (-OH) groups. An aliphatic compound having two hydroxyl groups is referred to as glycol or diol. A typical example of aliphatic compound having three hydroxyl groups is glycerol, and that of an aliphatic compound having four hydroxyl groups is pentaerythritol. Particularly, the polyol may be at least one selected from the group consisting of butylene glycol, propylene glycol, dipropylene glycol, erythritol, xylitol, sorbitol, polyethylene glycol and isoprene glycol, but is not limited thereto.

According to the present invention, the aqueous phase part of the water-in-oil type emulsion composition includes a salt. When a salt is incorporated to the aqueous phase part, it allows the chemically asymmetric anisotropic powder to be positioned between water and oil, and stabilizes the interface film to provide a stabilized water-in-oil type emulsion composition.

According to still another embodiment, the salt may be at least one selected from the group consisting of sodium chloride, potassium chloride, lithium chloride, calcium chloride and magnesium chloride.

According to still another embodiment, the salt may be present, for example, in an amount of at least 0.1 wt%, at least 0.2 wt%, at least 0.3 wt%, at least 0.4 wt%, at least 0.5 wt%, at least 0.6 wt%, at least 0.7 wt%, at least 0.8 wt%, at least 0.9 wt%, at least 1 wt%, at least 2 wt%, at least 3 wt%, at least 4 wt% or at least 5 wt%, and at most 10 wt%, at most 9 wt%, at most 8 wt%, at most 7 wt%, at most 6 wt% or at most 5 wt%, based on the total weight of the water-in-oil type emulsion composition. For example, the salt may be present in an amount of 0.1-10 wt% based on the total weight of the emulsion composition. It is possible to maintain the stability of the composition formulation against refrigeration/thawing and to provide excellent skin safety without any irritation within the above-defined range.

According to still another embodiment, the water-in-oil type emulsion composition may include the chemically asymmetric anisotropic powder, oil phase part and aqueous phase part at a mixing ratio of 1-15:50-80:10-30.

According to still another embodiment, the water-in-oil type emulsion composition may include emulsion particles having a size of 2-200 µm. In a variant, the water-in-oil type emulsion composition may include macroemulsion particles having a size of 10-100 µm, 10-50 µm, or 25 µm. Particularly, the water-in-oil type emulsion composition may include emulsion particles having a size of at least 2 µm, at least 5 µm, at least 10 µm, at least 15 µm, at least 20 µm, at least 25 µm, at least 30 µm, at least 40 µm, at least 50 µm, at least 80 µm, at least 100 µm, at least 130 µm, at least 150 µm or at least 180 µm, and at most 200 µm, at most 180 µm, at most 150 µm, at most 130 µm, at most 100 µm, at most 80 µm, at most 50 µm, at most 40 µm, at most 30 µm, at most 25 µm, at most 20 µm, at most 15 µm, at most 10 µm or at most 5 µm.

Since the chemically asymmetric anisotropic powder has different orientability against the interface, it is possible to form macroemulsion particles and to provide a formulation having an excellent feeling of use. It is difficult to form stabilized macroemulsion particles having a particle diameter of several tens of micrometers by using a molecular-level surfactant according to the related art, and the surfactant provides an interface film having a thickness of about several nanometers. However, in the case of the chemically asymmetric anisotropic powder disclosed herein, the thickness of the interface film increases to about several hundreds of nanometers and a stabilized interface film is formed by virtue of the strong binding among the powder particles, thereby improving emulsion stability significantly.

According to still another embodiment, the water-in-oil type emulsion composition may have a formulation with a low viscosity of 10000 cps or less. In the case of conventional W/O or W/S (silicon) formulations, most of them are provided as formulations having a viscosity higher than 10000 cps and a viscosity lower than 10000 cps causes a problem related with formulation stability. However, the water-in-oil type emulsion composition including the chemically asymmetric anisotropic powder not only has a formulation with a low viscosity of 1000 cps or less but also allows preparation of an emulsion composition having high stability.

According to yet another embodiment, the chemically asymmetric anisotropic powder may be added together with an aqueous phase part to provide a cosmetic emulsion composition.

The method for preparing chemically asymmetric anisotropic powder, includes:
(1) agitating a first monomer and a polymerization initiator to form a core of a first polymer spheroid; (2) agitating the formed core of a first polymer spheroid with a first monomer, a polymerization initiator and a functional group-containing compound to form a first polymer spheroid having a coated core-shell structure; and (3) agitating the formed first polymer spheroid having a core-shell structure with a second monomer and a polymerization initiator to obtain anisotropic powder in which a second polymer spheroid is formed.

In steps (1), (2) and (3), the agitation may be rotary agitation. Since homogeneous mechanical mixing is required together with chemical modification in order to produce uniform particles, rotary agitation is preferred. The rotary agitation may be carried out in a cylindrical reactor but is not limited thereto.

Herein, the internal design of the reactor significantly affects powder formation. The size and position of the baffles of the cylindrical reactor and the distance from an impeller have a significant effect upon the uniformity of the particles to be produced. Preferably, the interval between the internal blade and the blade of an impeller is minimized to make convection flow and intensity thereof uniform, the powdery reaction mixture is introduced to a level lower than the blade length, and the impeller is maintained at a high rotation speed. The rotation speed may be 200 rpm or higher, and the ratio of the diameter to the height of the reactor may be 1-3:1-5. Particularly, the reactor may have a diameter of 10-30 cm and a height of 10-50 cm. The reactor may have a size variable in proportion to the reaction capacity. In addition, the cylindrical reactor may be made of ceramics, glass or the like. The agitation is carried out preferably at a temperature of 50-90°C.

Simple mixing in a cylindrical rotary reactor allows production of uniform particles, requires low energy consumption and provides maximized reaction efficiency, and thus is amenable to mass production. The conventional tumbling method including rotation of a reactor itself causes inclination of the whole part of the reactor with a certain angle and rotation at a high speed, and thus requires high energy consumption and limits the reactor size. Due to such limitation in reactor size, the output is limited to a small amount of approximately several tens of milligrams to several grams. Thus, the conventional tumbling method is not suitable for mass production.

According to an embodiment, the first monomer and the second monomer may be the same or different, and particularly may be a vinyl monomer. In addition, the first monomer added in step (2) may be the same as the first monomer used in step (1) and the initiator used in each step may be the same or different.

According to another embodiment, the vinyl monomer may be a vinyl aromatic monomer. The vinyl aromatic monomer may be substituted or non-substituted styrene, such as at least one selected from the group consisting of styrene, alpha-methylstyrene, alpha-ethylstyrene and para-methylstyrene.

According to still another embodiment, the polymerization initiator may be a radical polymerization initiator. Particularly, the polymerization initiator may be at least one selected from peroxide-based and azo-based initiators. In addition, ammonium persulfate, sodium persulfate or potassium persulfate may be used. The peroxide-based radical polymerization initiator may be at least one selected from the group consisting of benzoyl peroxide, lauryl peroxide, cumene hydroperoxide, methylethyl ketone peroxide, t-butyl hydroperoxide, o-chlorobenzoyl peroxide, o-methoxylbenzoyl peroxide, t-butylperoxy-2-ethylhexanoate and t-butylperoxy isobutyrate. The azo-based radical polymerization initiator may be at least one selected from the group consisting of 2,2'-azobisisobutyronitrile, 2,2'-azobis(2-methylisobutyronitrile) and 2,2'-azobis(2,4-dimethylvaleronitrile).

According to still another embodiment, in step (1), the first monomer and the polymerization initiator may be mixed at a weight ratio of 100-250:1.

In a variant, in step (1), a stabilizer is added together with the first monomer and the polymerization initiator in such a manner that the first monomer, polymerization initiator and the stabilizer may be mixed at a weight ratio of 100-250:1:0.001-5. The size and shape of the powder is determined by controlling the size of the first polymer spheroid in step (1), and the size of the first polymer spheroid may be controlled by the ratio of the first monomer, initiator and the stabilizer. In addition, it is possible to increase the uniformity of anisotropic powder by mixing the first monomer, polymerization initiator and the stabilizer within the above-defined weight ratio.

According to an embodiment, the stabilizer may be an ionic vinyl monomer, and particularly sodium 4-vinylbenzene sulfonate may be used. The stabilizer prevents swelling of the particles, and imparts positive or negative charges to the powder surface, thereby preventing coalescence (binding) of the particles electrostatically.

When the chemically anisotropic powder has a size of 200-250 nm, it may be obtained from the first polymer spheroid including the first monomer, initiator and the stabilizer at a ratio of 110-130:1:2-4, particularly 115-125:1:2-4, and more particularly 120:1:3.

In addition, when the chemically anisotropic powder has a size of 400-450 nm, it may be obtained from the first polymer spheroid including the first monomer, initiator and the stabilizer at a ratio of 225-240:1:1-3, particularly 230-235:1:1-3, and more particularly 235:1:2.

Further, when the chemically anisotropic powder has a size of 1100-1500 nm, it may be obtained from the first polymer spheroid including the first monomer, initiator and the stabilizer at a ratio of 110-130:1:0, particularly 115-125:1:0, and more particularly 120:1:0.

In addition, chemically anisotropic powder having an asymmetric snowman shape may be obtained from the first polymer spheroid prepared by reacting the first monomer, initiator and the stabilizer at a ratio of 100-140:1:8-12, particularly 110-130:1:9-11, and more particularly 120:1:10.

Further, chemically anisotropic powder having an asymmetric reverse snowman shape may be obtained from the first polymer spheroid prepared by reacting the first monomer, initiator and the stabilizer at a ratio of 100-140:1:1-5, particularly 110-130:1:2-4, and more particularly 120:1:3.

According to another embodiment, in step (2), the functional group-containing monomer may be a siloxane-containing compound. Particularly, the functional group-containing monomer may be a siloxane-containing (meth)acrylate polymer, and may be at least one selected from the group consisting of 3-(trimethoxysilyl)propyl acrylate, 3-(trimethoxysilyl)propyl methacrylate, vinyltriethoxysilane and vinyltrimethoxysilane.

According to still another embodiment, in step (2), the first monomer, polymerization initiator and the functional group-containing monomer may be mixed at a weight ratio of 80-98:0.2-0.8:2-20. In a variant, the first monomer, polymerization initiator and the functional group-containing monomer may be mixed at a weight ratio of 160-200:1:6-40. It is possible to control the coating degree according to the reaction ratio, and then the coating degree determines the shape of amphiphilic anisotropic powder. When the first monomer, polymerization initiator and the functional group-containing compound are used within the above-defined ratio, the coating thickness is increased by about 10-30%, particularly approximately 20%, based on the initial thickness. In this case, formation of powder proceeds smoothly without problems, such as a failure in formation of powder caused by excessively thick coating or multi-directional formation of powder caused by excessively thin coating. In addition, it is possible to increase the uniformity of anisotropic powder within the above weight ratio.

According to still another embodiment, in step (3), the second monomer and polymerization initiator may be mixed at a weight ratio of 200-250:1.

In a variant, in step (3), a stabilizer may be added together with the second monomer and polymerization initiator in such a manner that the second monomer, polymerization initiator and the stabilizer may be mixed at a weight ratio of 200-250:1:0.001-5. Particular examples of the stabilizer are the same as described above. It is possible to increase the uniformity of anisotropic powder within the above weight ratio.

According to still another embodiment, in step (3), the second monomer may be mixed in an amount of 40-100 parts by weight or 150-300 parts by weight based on 100 parts by weight of the first polymer spheroid having a core-shell structure. Particularly, when the content of the second monomer is 40-100% based on the weight of the first polymer spheroid, asymmetric snowman-like powder is obtained. When the content of the second monomer is 100-150% or 110-150% based on the weight of the first polymer spheroid, symmetric powder is obtained. In addition, when the content of the second monomer is 150-300% or 160-300% based on the weight of the first polymer spheroid, asymmetric reverse snowman-like powder is obtained. It is possible to increase the uniformity of anisotropic powder within the above weight ratio.

According to the related art, many attempts have been made to increase the surface active property of spherical powder particles used for Pickering by imparting amphiphilic surface active property thereto. This may be exemplified by Janus spherical particles. However, such particles are geometrically limited and have a problem in terms of uniform mass production, and thus cannot be applied practically. On the contrary, the method for preparing chemically anisotropic powder disclosed herein uses no crosslinking agent, thereby causing no agglomeration and providing high yield and uniformity. In addition, the method disclosed herein uses a simple agitation process and is more amenable to mass production as compared to a tumbling process. Particularly, the method disclosed herein is advantageous in that it allows production of nano-size particles having a size of 300 nm or less in a large scale of several tens of grams and several tens of kilograms.

According to still another embodiment, the method for preparing chemically asymmetric anisotropic powder may further include step (4), i.e., introducing a hydrophilic functional group to the anisotropic powder after step (3).

According to still another embodiment, in step (4), hydrophilic functional group may be introduced by using a silane coupling agent and reaction modifier, but is not limited thereto.

According to still another embodiment, the silane coupling agent may be at least one selected from the group consisting of (3-aminopropyl)trimethoxysilane, N-[3-(trimethoxysilyl)propyl]ethylenediamine, N-[3-(trimethoxysilyl)propyl]ethylenediammonium chloride, (N-succinyl-3-aminopropyl)trimethoxysilane, 1-[3-(trimethoxysilyl)propyl]urea and 3-[(trimethoxysilyl)propyloxy]-1,2-propanediol. Particularly, the silane coupling agent may be N-[3-(trimethoxysilyl)propyl]ethylene diamine.

According to still another embodiment, the silane coupling agent may be incorporated in an amount of 35-65 parts by weight, such as 40-60 parts by weight, based on 100 parts by weight of the anisotropic powder obtained from step (3). It is possible to carry out hydrophilization adequately within the above-defined range.

According to still another embodiment, the reaction modifier may be ammonium hydroxide.

According to yet another embodiment, the reaction modifier may be incorporated in an amount of 85-115 parts by weight, such as 90-110 parts by weight, based on 100 parts by weight of the anisotropic powder obtained from step (3). It is possible to carry out hydrophilization adequately within the above-defined range.

### [Mode for Invention]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein.

### Preparation Example 1. Preparation of Polystyrene (PS) First Polymer Spheroid

Styrene as a monomer, sodium 4-vinylbenzene sulfonate as a stabilizer and azobisisobutyronitrile (AIBN) as an initiator are mixed in an aqueous phase and are allowed to react at 75°C for 8 hours. The reaction is carried out by agitating the reaction mixture in a cylindrical reactor having a diameter of 11 cm and a height of 17 cm and made of glass under a speed of 200 rpm.

### Preparation Example 2. Preparation of Coated First Polymer Spheroid Having Core-Shell (CS) Structure

The polystyrene (PS) first polymer spherical particles obtained as described above are mixed with styrene as a monomer, 3-(trimethoxysilyl)propyl acrylate (TMSPA) and azobisisobutyronitrile (AIBN) as an initiator and the reaction mixture is allowed to react. The reaction is carried out by agitating the reaction mixture in a cylindrical reactor.

### Preparation Example 3. Preparation of Anisotropic Powder

The aqueous dispersion of the polystyrene-core shell (PC-CS) dispersion obtained as described above is mixed with styrene as a monomer, sodium 4-vinylbenzene sulfonate as a stabilizer and azobisisobutyronitrile (AIBN) as an initiator and the reaction mixture is heated to 75°C to carry out reaction. The reaction is carried out by agitating the reaction mixture in a cylindrical reactor. In this manner, amphiphilic anisotropic powder is obtained.

### Preparation Example 4. Preparation of Hydrophilized Amphiphilic Anisotropic Powder

To 600 g of the aqueous dispersion of the anisotropic powder obtained as described above, 30 g of N-[3-(trimethoxysilyl)propyl]ethylenediamine as a silane coupling agent and 60 g of ammonium hydroxide as a reaction modifier are added, followed by mixing, to carry out reaction at 25°C for 24 hours so that a hydrophilic functional group is introduced. The reaction is carried out by agitating the reaction mixture in a cylindrical reactor. In this manner, hydrophilized amphiphilic anisotropic powder is obtained.

### Examples and Comparative Examples

The anisotropic powder obtained from Preparation Example 3 is used to obtain the water-in-oil type emulsion compositions as shown in the following Table 1.

**[Table 1]**

| (Unit: wt%) | Example 1 | Example 2 | Comparative Example 1 |
|---|---|---|---|
| Silicon (Cyclopentasiloxane) | 30 | 0 | 15 |
| Oil (Squalane) | 0 | 30 | 5 |
| Inorganic thickener (Disteardimonium Hectorite) | 0.2 | 0.2 | 0.5 |
| Oil (Diethoxyethyl succinate) | 2 | 2 | 0 |
| Silicon oil (PCA Dimethicone) | 5 | 5 | 0 |
| Purified water (D.I.Water) | to 100 | to 100 | to 100 |
| Salt (NaCl) | 2 | 2 | 1 |
| Dispersion of anisotropic powder (20 wt% in ethanol) | 15 | 15 | 0 |
| Surfactant (Cyclopentasiloxane*PEG-10 Dimethicone* Disteardimonium Hectorite) | 0 | 0 | 4 |
| Surfactant (PEG-10 Dimethicone) | 0 | 0 | 2 |
| Surfactant (Lauryl PEG-9 Polyd imethylsiloxyethyl Dimethicone) | 0 | 0 | 0.5 |

### Test Example 1. Comparison of Viscosity of Emulsion Composition

To determine the stability of an emulsion composition including anisotropic powder, each of the emulsion composition according to Example 1 and the conventional emulsion composition according to Comparative Example 1 is dropped onto a black-colored square plastic plate in an amount of about 1.5 g. Then, the plate is allowed to stand at an angle of 45° from the ground surface for 15 minutes to carry out comparison of viscosity. The results are shown in the image of Fig. 1.

As shown in Fig. 1, the W/O formulation including anisotropic powder according to Example 1 shows a controlled size of emulsion particles and viscosity of outer phase, unlike the conventional W/O formulation according to Comparative Example 1. It can be seen that a low-viscosity formulation is obtained. It is difficult to obtain such low viscosity from the conventional water-in-oil type formulation due to its emulsion stability. On the contrary, the composition according to Example 1 has low viscosity so that the emulsion particles cause no coalescence and maintain a stable emulsion state.

### Test Example 2. Evaluation of Temperature Stability of Emulsion Composition

The composition according to Example 1 is stored under refrigeration at -15°C to -25°C for 2 months. In addition, the composition is maintained at - 20°C under refrigeration for at least 1 week, and then is stored for 2 months while it is thawed four times repeatedly to observe the stability of emulsion particles as a function of temperature. After the observation, the optical microscopic images are shown in Fig. 2.

As shown in Fig. 2, the water-in-oil type emulsion composition has stable emulsion particles not only right after its preparation at room temperature (25°C) (portion (a) of Fig. 2) but also after storing it for 2 months under refrigeration (portion (b) of Fig. 2) and after repeating refrigeration and thawing four times (portion (c) of Fig. 2).

### Test Example 3. Evaluation of Stability of Emulsion Composition with Time

While the composition according to Example 1 is maintained at 30°C for 12 weeks, it is determined for variations in viscosity by using a viscometer (LVDV-II+PRO, BROOKFIELD, USA). The results are shown in Fig. 3.

It can be seen from Fig. 3 that the viscosity of the composition converges on a constant viscosity with no significant change with time, which demonstrates that the composition has excellent stability with time.

While the exemplary embodiments have been shown and described, it will be understood by those skilled in the art that various changes in form and details may be made thereto without departing from the disclosure as defined by the appended claims. Therefore, it is intended that the scope of the present disclosure includes all embodiments falling within the disclosure of the appended claims.

## Claims

1. A water-in-oil (W/O) type emulsion comprising chemically asymmetric anisotropic powder which comprises a first hydrophilic polymer spheroid and a second hydrophobic polymer spheroid, wherein the first and second polymer spheroids are bound to each other with a structure in which one polymer spheroid at least partially infiltrates the other polymer spheroid; the first polymer spheroid has a core-shell structure; and the shell has a functional group,
wherein the second polymer spheroid and the core of the first polymer spheroid comprise a vinyl polymer, and the shell of the first polymer spheroid comprises a copolymer of a vinyl monomer with a functional group-containing monomer which is siloxane;
and the vinyl polymer is polystyrene, and
wherein the aqueous phase part of the water-in-oil type emulsion composition comprises a salt.

2. The water-in-oil (W/O) type emulsion composition according to claim 1, wherein the powder has an asymmetric snowman shape or asymmetric reverse snowman shape.

3. The water-in-oil (W/O) type emulsion composition according to claim 1, wherein the chemically asymmetric anisotropic powder is present in an amount of 1-15 wt% based on the total weight of the emulsion composition.

4. The water-in-oil (W/O) type emulsion composition according to claim 1, which comprises an alcohol.

5. The water-in-oil (W/O) type emulsion composition according to claim 1, wherein the salt is at least one selected from the group consisting of sodium chloride, potassium chloride, lithium chloride, calcium chloride and magnesium chloride.

6. The water-in-oil (W/O) type emulsion composition according to claim 1, wherein the salt is present in an amount of 0.1-10 wt% based on the total weight of the water-in-oil type emulsion composition.

7. The water-in-oil (W/O) type emulsion composition according to claim 1, which comprises the chemically asymmetric anisotropic powder, oil phase part and aqueous phase part at a mixing ratio of 1-15:50-80:10-30 on the weight basis.

8. The water-in-oil (W/O) type emulsion composition according to claim 1, which has emulsion particles having a size of 2-200 µm.

9. The water-in-oil (W/O) type emulsion composition according to claim 1, which has a formulation with a low viscosity of 10000 cps or less.

## Patentansprüche

1. Emulsion vom Typ Wasser-in-ÖI (W/O), die chemisch asymmetrisches anisotropes Pulver umfasst, das eine erste hydrophile Polymerkugel und eine zweite hydrophobe Polymerkugel umfasst, wobei die erste und zweite Polymerkugel mit einer Struktur aneinander gebunden sind, in der eine Polymerkugel die andere Polymerkugel mindestens teilweise infiltriert; die erste Polymerkugel eine Kern-Schale-Struktur aufweist; und die Schale eine funktionelle Gruppe aufweist,
wobei die zweite Polymerkugel und der Kern der ersten Polymerkugel ein Vinylpolymer umfassen, und die Schale der ersten Polymerkugel ein Copolymer aus einem Vinylmonomer mit einem Monomer umfasst, das eine funktionelle Gruppe enthält, die Siloxan ist;
und das Vinylpolymer Polystyrol ist, und
wobei der Wasserphasen-Teil der Emulsionszusammensetzung vom Typ Wasser-in-ÖI ein Salz umfasst.

2. Emulsionszusammensetzung vom Typ Wasser-in-ÖI (W/O) nach Anspruch 1, wobei das Pulver eine asymmetrische Schneemannform oder asymmetrische umgekehrte Schneemannform aufweist.

3. Emulsionszusammensetzung vom Typ Wasser-in-ÖI (W/O) nach Anspruch 1, wobei das chemisch asymmetrische anisotrope Pulver auf Basis des Gesamtgewichts der Emulsionszusammensetzung in einer Menge von 1-15 Gew.-% vorliegt.

4. Emulsionszusammensetzung vom Typ Wasser-in-ÖI (W/O) nach Anspruch 1, die einen Alkohol umfasst.

5. Emulsionszusammensetzung vom Typ Wasser-in-ÖI (W/O) nach Anspruch 1, wobei das Salz mindestens eines ist, ausgewählt aus der Gruppe bestehend aus Natriumchlorid, Kaliumchlorid, Lithiumchlorid, Calciumchlorid und Magnesiumchlorid.

6. Emulsionszusammensetzung vom Typ Wasser-in-ÖI (W/O) nach Anspruch 1, wobei das Salz auf Basis des Gesamtgewichts der Emulsionszusammensetzung vom Typ Wasser-in-ÖI in einer Menge von 0,1-10 Gew.-% vorliegt.

7. Emulsionszusammensetzung vom Typ Wasser-in-ÖI (W/O) nach Anspruch 1, die das chemisch asymmetrische anisotrope Pulver, den Ölphasen-Teil und den Wasserphasen-Teil in einem Mischungsverhältnis, auf Gewichtsbasis, von 1-15:50-80:10-30 umfasst.

8. Emulsionszusammensetzung vom Typ Wasser-in-ÖI (W/O) nach Anspruch 1, die Emulsionsteilchen aufweist, die eine Größe von 2-200 µm aufweisen.

9. Emulsionszusammensetzung vom Typ Wasser-in-ÖI (W/O) nach Anspruch 1, die eine Formulierung mit einer niedrigen Viskosität von 10 000 cps oder weniger aufweist.

## Revendications

1. Émulsion de type eau dans l'huile (E/H) comprenant une poudre anisotrope chimiquement asymétrique qui comprend un premier sphéroïde polymère hydrophile et un deuxième sphéroïde polymère hydrophobe, où les premier et deuxième sphéroïdes polymères sont liés l'un à l'autre avec une structure dans laquelle un sphéroïde polymère s'infiltre au moins partiellement dans l'autre sphéroïde polymère ; le premier sphéroïde polymère a une structure noyau-enveloppe ; et l'enveloppe a un groupe fonctionnel,
dans laquelle le deuxième sphéroïde polymère et le noyau du premier sphéroïde polymère comprennent un polymère vinylique et l'enveloppe du premier sphéroïde polymère comprend un copolymère d'un monomère vinylique avec un monomère contenant un groupe fonctionnel qui est le siloxane ;
et le polymère vinylique est un polystyrène, et
dans laquelle la partie de phase aqueuse de la composition d'émulsion de type eau dans l'huile comprend un sel.

2. Composition d'émulsion de type eau dans l'huile (E/H) selon la revendication 1, dans laquelle la poudre a la forme d'un bonhomme de neige asymétrique ou la forme d'un bonhomme de neige inversé asymétrique.

3. Composition d'émulsion de type eau dans l'huile (E/H) selon la revendication 1, dans laquelle la poudre anisotrope chimiquement asymétrique est présente en une quantité allant de 1 à 15% en poids par rapport au poids total de la composition d'émulsion.

4. Composition d'émulsion de type eau dans l'huile (E/H) selon la revendication 1, qui comprend un alcool.

5. Composition d'émulsion de type eau dans l'huile (E/H) selon la revendication 1, dans laquelle le sel est au moins un élément choisi dans le groupe constitué de chlorure de sodium, de chlorure de potassium, de chlorure de lithium, de chlorure de calcium et de chlorure de magnésium.

6. Composition d'émulsion de type eau dans l'huile (E/H) selon la revendication 1, dans laquelle le sel est présent en une quantité allant de 0,1 à 10% en poids par rapport au poids total de la composition d'émulsion de type eau dans l'huile.

7. Composition d'émulsion de type eau dans l'huile (E/H) selon la revendication 1, qui comprend la poudre anisotrope chimiquement asymétrique, une partie de phase huileuse et une partie de phase aqueuse à un rapport de mélange de 1-15 : 50-80 : 10-30 en poids.

8. Composition d'émulsion de type eau dans l'huile (E/H) selon la revendication 1, qui a des particules d'émulsion ayant une taille allant de 2 à 200 µm.

9. Composition d'émulsion de type eau dans l'huile (E/H) selon la revendication 1, qui a une formulation ayant une faible viscosité inférieure ou égale à 10000 cps.
